# FASCICULE DE BREVET EUROPEEN

(11) **EP 0 871 611 B1**
(45) Date de publication et mention de la délivrance du brevet: **13.09.2000**
(21) Numéro de dépôt: 96901411.7
(22) Date de dépôt: 18.01.1996
(51) Int. Cl.: C07C 323/60, C07C 237/22, A61K 31/195

(54) **NOUVEAUX DERIVES DE GLYCYLANILIDES, LEUR PREPARATION ET LEUR APPLICATION EN THERAPEUTIQUE**
NEUE GYLCYLANILID-DERIVATE, DEREN HERSTELLUNG UND DEREN VERWENDUNG ZUR BEHANDLUNG VON HYPERCHOLESTEROLÄMIE UND ATHEROSKLEROSE
NEW DERIVATIVES OF GLYCYLANILIDES, PREPARATION AND THERAPEUTICAL APPLICATION

(30) Priorité: 19.01.1995 FR 9500577
(43) Date de publication de la demande: 21.10.1998
(73) Titulaire: PIERRE FABRE MEDICAMENT, 92100 Boulogne (FR)
(72) Inventeur: PATOISEAU, Jean-François, F-81100 Castres (FR); AUTIN, Jean-Marie, F-81290 Labruguiere (FR); DELHON, André, F-81100 Castres (FR); OMS, Philippe, F-81100 Castres (FR)
(74) Mandataire: Warcoin, Jacques
(86) Numéro de dépôt international: FR9600081
(87) Numéro de publication internationale: WO9622279

(56) Documents cités:
- EP-A- 0 415 413
- EP-A- 0 559 898
- EP-A- 0 587 430

## Description

La présente invention a pour objet de nouveaux dérivés de glycylanilides, leur préparation et leur application en thérapeutique humaine. Elle concerne également l'utilisation de ces dérivés pour la fabrication de médicaments destinés au traitement de l'hypercholestérolémie ou de l'athérosclérose.

Le cholestérol alimentaire est absorbé sous forme de cholestérol libre par les cellules intestinales, estérifié par l'enzyme ACAT (acyl CoA : cholestérol O-Acyl transférase), dans le sérum. L'inhibition de l'ACAT prévient l'absorption intestinale et l'accumulation de cholestérol dans le tissu artériel. En outre, les lipoprotéines de basse densité (LDL) sont, après oxydation, captées par les "scavenger receptors" et conduisent à la formation de la cellule spumeuse, point de départ de la plaque d'athérome (D. STEINBERG et al. England. J. Med. 320, 915-924, 1989).

L'objet de la présente invention vise à obtenir de nouveaux dérivés hypocholestérolémiants et antioxydants pouvant agir à la fois sur la qualité et la quantité des LDL dans le but de réduire leur potentiel athérogène et leurs effets délétères à long terme sur la paroi vasculaire.

Les composés de la présente invention répondent à la formule générale I. 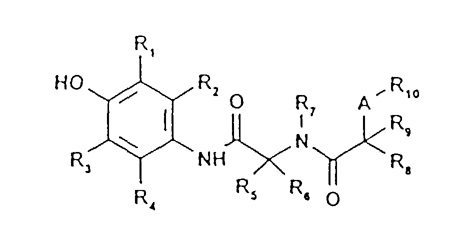 dans laquelle
- R₁, R₂, R₃, R₄, R₇, identiques ou différents représentent indépendamment les uns des autres l'hydrogène ou un radical alcoyle linéaire ou ramifié en C₁-C₄, à la condition toutefois qu'au moins trois des substituants R_{1,} R₂, R₃ et R₄ soient différents d'un atome d'hydrogène.
- R₅, R₆, R₈, et R₉, identiques ou différents représentent indépendamment les uns des autres l'hydrogène, un radical alcoyle linéaire ou ramifié en C₁-C₄, ou un radical phényle éventuellement substitué.
- R₁₀ représente un radical alcoyle linéaire ou ramifié en C₃-C₁₆.
- A représente un atome de soufre ou un groupement méthylène.

Les composés de formule générale I pouvant posséder des centres asymétriques, la présente invention couvre tous les stéréoisomères et leurs mélanges.

Les composés de formule générale I peuvent être préparés selon la méthode suivante (schéma 1).

### Etape 1:

Traitement d'une aniline par un amino acide protégé au niveau de la fonction amine.
. Le groupement protecteur peut être le groupement t-butyloxy carbonyl (Boc).
. Le couplage avec l'aniline peut s'effecteur par traitement au chloroformate d'éthyle dans le diméthyl formamide en présence de triéthylamine.
. La déprotection peut s'effectuer en milieu acide chlorhydrique 6N dans le méthanol.

On obtient ainsi le composé II.

### Etape 2a :

Traitement du composé II obtenu à l'étape 1 par un chlorure d'acide III pour obtenir les composés I dans lesquels A = CH₂ ou par un chlorure d'acide a halogéné puis par un thiol R₁₀SH en milieu sodium éthanol ou en milieu tertiobutylate de potassium dans le tertiobutanol, pour obtenir les composés I dans lesquels A = soufre.

### Etape 2b :

Traitement du composé II par un chlorure d'acide IV dans l'acétat d'éthyle en présence de triéthylamine pour obtenir des composés de formule I dans lesquels A est un atome de soufre ou un groupement méthylène.

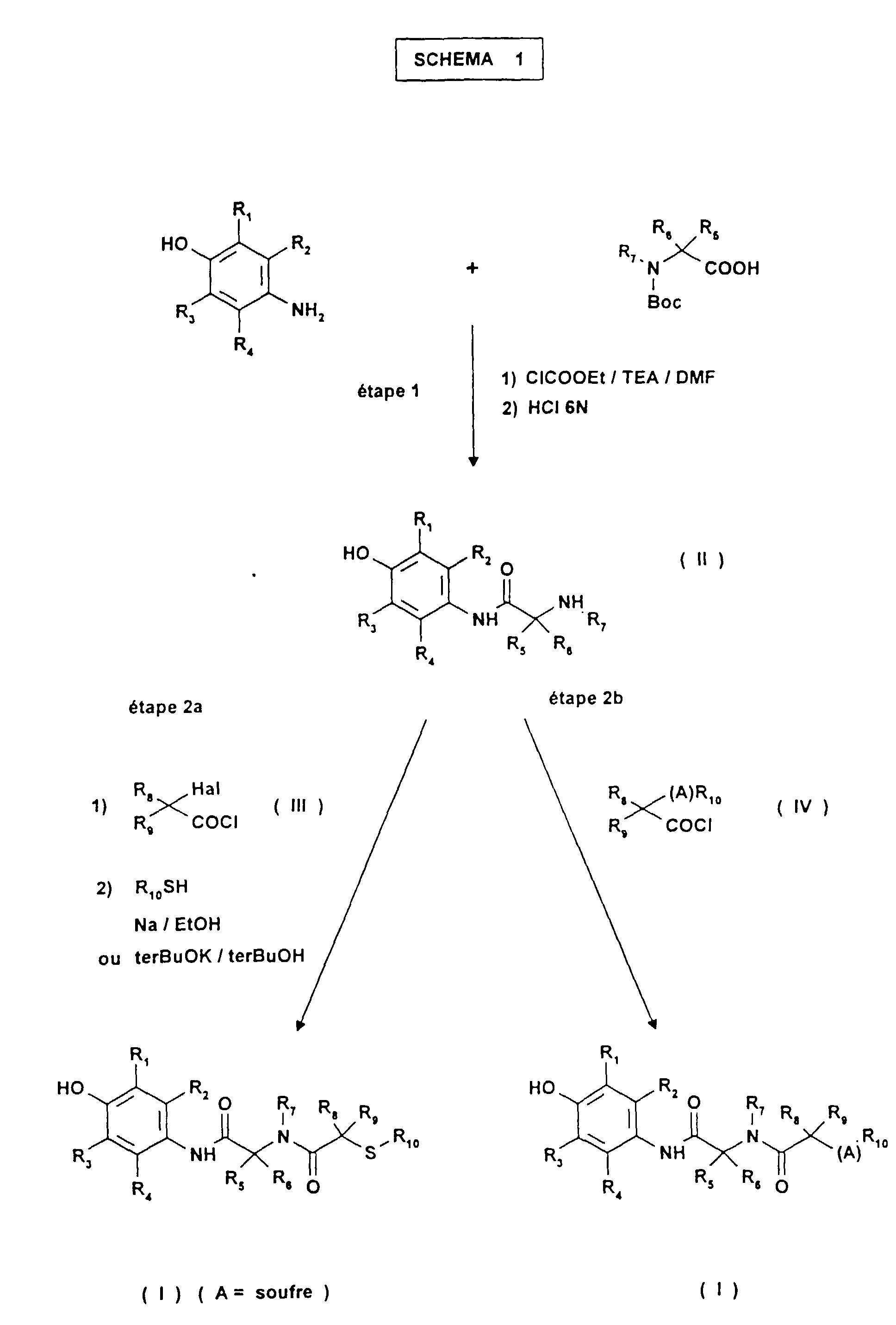

Les composés de formule générale I peuvent être utilisés pour la préparation de compositions pharmaceutiques ou de médicaments destinés au traitement de maladies telles que l'hypercholestérolémie ou l'athérosclérose.

L'invention pourra être mieux comprise à l'aide des exemples 1 à 11 non limitatifs qui suivent et qui constituent des modes de réalisation avantageux du procédé selon l'invention. Les exemples 12 à 17 ne sont cités qu'à titre comparatif, mais n'appartiennent pas à l'invention.

### EXEMPLE 1 :

**N-(2-n-dodécylthio) propionamido-2',3',5'-triméthyl-4'-hydroxyglycylanilide 1.** 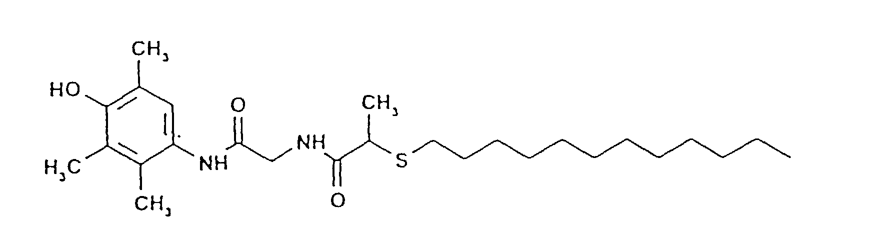
**a) N-tertiobutoxycarbonyl-2',3',5'-triméthyl-4'-hydroxy-glycylanilide 1a.**
   A une solution glacée de boc-glycine (1,75 g ; 0,01 mole) et de N-méthylmorpholine (2,19 ml ; 0,02 mole) dans le chlorure de méthylène (10 ml) est ajoutée goutte à goutte une solution de chloroformate d'éthyle (0,96 ml ; 0,01 mole) dans le CH₂Cl₂ (5 ml). Après 20 minutes de contact à 0°C, on ajoute sous azote du chlorhydrate de 2,3,6-triméthyl-4-aminophénol (1,88 g ; 0,01 mole) dans le CH₂Cl₂ (40 ml). Le mélange réactionnel est ensuite agité 24 heures à température ambiante et évaporé sous vide. Le résidu est repris à l'eau, extrait à l'acétate d'éthyle. La phase organique est lavée à l'acide chlorhydrique N et à l'eau puis séchée (Na₂SO₄) et concentrée sous vide. Le résidu est purifié par chromatographie flash. On obtient 1,42 g de cristaux 1a.
   F = 208°C
   CCM : gel de silice 60F254 Merck. Rf = 0,54 (AcOEt)
**b) 2',3',5'- triméthyl-4'-hydroxy-glycylanilide 1b.**
   12 ml d'acide chlorhydrique 6N sont ajoutés au composé la (3,08 g ; 0,01 mole) dans le méthanol (100 ml). La solution est agitée à 60°C pendant 5 heures puis concentrée sous vide. Le solide est repris à l'eau, amené à pH 9 par addition de soude N et extrait 3 fois à l'acétate d'éthyle. Les phases organiques réunies sont lavées à l'eau, séchées (Na₂SO₄) et évaporées sous vide. Un empâtage dans l'hexane fournit après filtration le composé 1b (1,89g).
   F = 177°C
   CCM : gel de silice 60F254 Merck. Rf = 0,30 (CH₂ Cl₂ - MeOH 50-50)
**c) N-α bromopropionamido-2',3',5'-triméthyl-4'-hydroxy-glycylanilide 1c.**
   Une solution de dérivé 1b (2,08 g ; 0,01 mole) dans l'acétate d'éthyle (35 ml) est additionnée de triéthylamine (1,67 ml ; 0,012 mole) puis, goutte à goutte, de bromure d'α-bromopropionyle dans l'acétate d'éthyle (15 ml) à température ambiante. Au bout d'une heure d'agitation, le milieu réactionnel est lavé à l'eau, séché (Na₂SO₄) et concentré sous vide. Le solide est repris à l'hexane, filtré et séché pour donner le composé 1c (2,89 g).
   F = 178°C
   CCM : Gel de silice 60F254 Merck. Rf = 0,48 (CH₂Cl₂- MeOH 90-10).
**d) N-(2-n-dodécylthio) propionamido-2',3',5'-triméthyl-4'-hydroxyglycylanilide 1.**
   Un mélange de sodium à 45 % dans la paraffine (0,63 g, 0,012 at. g.) et de n-dodécanethiol (2,9 ml ; 0,01 mole) est chauffé 45 minutes à 110°C. Après refroidissement, le thiolate est dissous dans l'éthanol (30 ml). Le dérivé lc (3,43 g ; 0,01 mole) est alors ajouté et le tout est porté à reflux pendant 6 heures. Le mélange réactionnel est évaporé sous vide et l'huile résiduelle extraite au chlorure de méthylène. La phase organique est lavée à HCl N, à leau et à l'eau salée. Après séchage et concentration de la solution, on obtient des cristaux blancs de produit 1 (3,29 g).
   F = 109°C
   CCM : gel de silice 60F254 Merck. Rf = 0.33 (CH₂Cl₂- AcOEt 70-30)

### EXEMPLE 2 :

### N-(α,α,-diméthyl)tétradécanamido-2',3',5'-triméthyl-4'hydroxyglycylanilide.

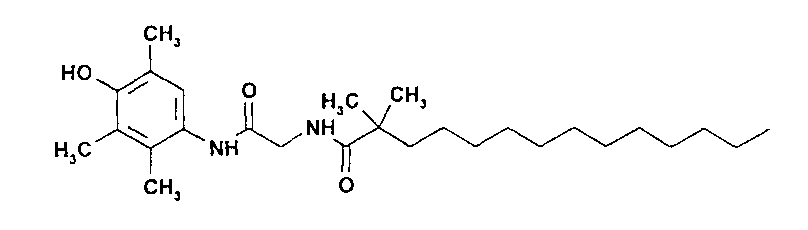

A une solution de 2',3',5'-triméthyl-4'-hydroxy-glycylanilide (2,08 g; 0,01 mole) (préparé selon 1b) et de triéthylamine (3,34 ml ; 0,024 ml) dans le DMF (20 ml), on ajoute goutte à goutte du chlorure d'α,α-diméthyltétradécanoyle (3,3 g ; 0,01 mole, préparé par action de chlorure de thionyle sur l'acide correspondant 3 heures au reflux du toluène) dans le tétrahydrofurane (40 ml). Après 4 heures d'agitation à température ambiante, le mélange réactionnel est dilué à l'eau et extrait à l'acétate d'éthyle. La phase organique est lavée à l'HCl N et à l'eau puis séchée et concentrée sous vide. Le solide obtenu est purifié par chromatographie flash (3,13 g).
F = 162°C
CCM : gel de silice 60F254 Merck. Rf = 0,56 (AcOEt)

### EXEMPLE 3 :

### N-méthyl-N-(α-n-dodécylthio)isobutyramido-2',3',5'-triméthyl-4'-hydroxy-glycylanilide.

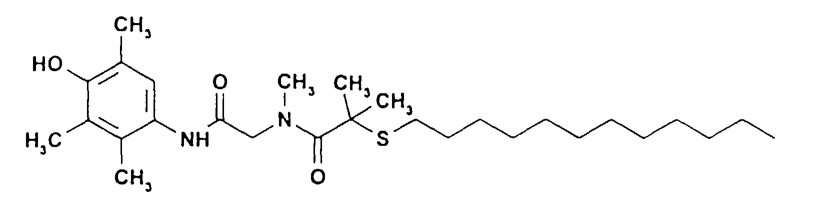

Ce composé a été préparé selon le procédé décrit à l'exemple 1a et 1b en utilisant la N-boc-sarcosine, puis condensation du chlorure d'α-n-dodécylthioisobutyryle selon l'exemple 2.
F = 115°C
CCM : gel de silice 60F254 Merck. Rf = 0,46 (AcOEt - Hexane 50-50).

### EXEMPLE 4 :

### N-(α-n-dodécylthio)-butyramido-2',3',5'-triméthyl-4'-hydroxyglycylanilide.

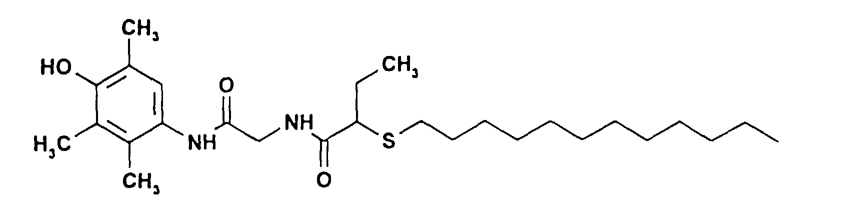

Ce composé a été préparé selon le procédé décrit à l'exemple 1, en utilisant le bromure de 2-bromobutyryle.
F = 115°C
CCM : gel de silice 60F254 Merck. Rf = 0,46 (AcOEt - Hexane 50-50).

### EXEMPLE 5 :

### N-(α-n-dodécylthio) isobutyramido-2',3',5'-triméthyl-4'-hydroxyglycylanilide.

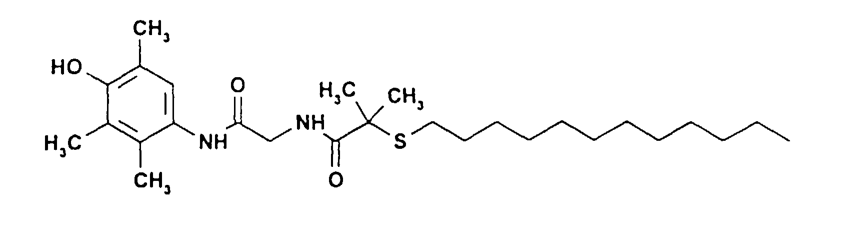

Ce composé a été préparé selon le procédé décrit à l'exemple 2 en utilisant le chlorure d'α-dodécylthio-isobutyryle.
F = 113°C
CCM : gel de silice 60F254 Merck. Rf = 0,37 (AcOEt - Hexane 50-50).

### EXEMPLE 6 :

### N-(d-n-dodécylthio) acétamido-2',3',5'-triméthyl-4'-hydroxyglycylanilide.

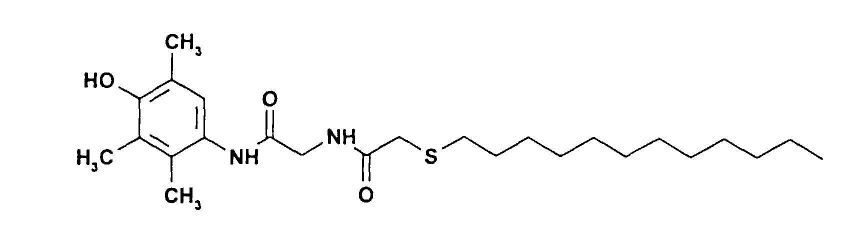

Ce composé a été préparé selon le procédé décrit à l'exemple 1 en utilisant le chlorure de chloracétyle.
F = 128°C
CCM : gel de silice 60F254 Merck. Rf = 0,50 (AcOEt).

### EXEMPLE 7 :

### N-(α-n-dodécylthio)butyramido-2-éthyl-2',3',5'-triméthyl-4'-hydroxyglycylanilide.

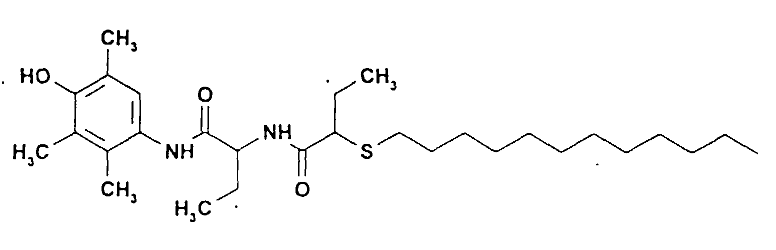

Ce composé a été préparé selon les procédés décrits dans les exemples 1a et 1b en utilisant l'acide α-bocamino-butyrique puis les procédés 1c et 1d en utilisant le bromure d'α-bromobutyryle..
F = 153°C
CCM = gel de silice 60F254 Merck. Rf = 0,54 (CH₂Cl₂- AcOEt 70-30).

### EXEMPLE 8 :

### N-méthyl-N-(α-n-dodécylthio)propionamido-2',3',5'-triméthyl-4'-hydroxyglycylanilide.

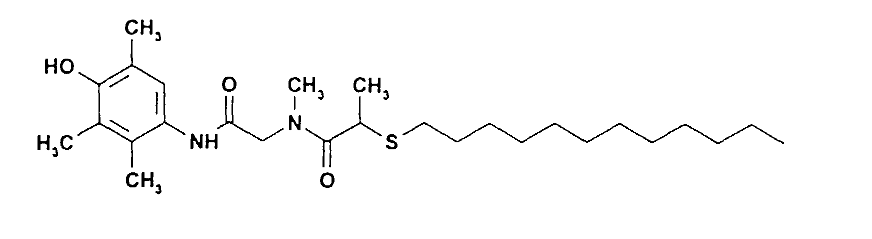

Ce composé a été préparé selon les procédés décrits dans les exemples 1a et 1b en utilisant la boc-sarcosine puis les procédés 1c et 1d en utilisant le bromure de 2-bromopropionyle.
F = 106°C
CCM = gel de silice 60F254 Merck. Rf = 0,47 (AcOEt).

### EXEMPLE 9 :

### N-(α-n-dodécylthio)isobutyramido-2-n-butyl-2',3',5'-triméthyl-4'-hydroxy-glycylanilide.

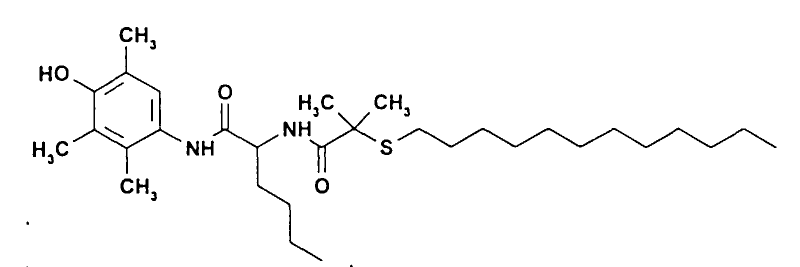

Ce composé a été préparé selon les procédés décrits dans les exemples 1a et 1b en utilisant l'acide α-amino-hexanoïque selon le procédé de l'exemple 2 en utilisant le chlorure d'α-dodécylthio-isobutyryle.
F = 113°C
CCM : gel de silice 60F254 Merck. Rf = 0,40 (CH₂Cl₂- AcOEt 90-10).

### EXEMPLE 10 :

### N-(α-n-dodécylthio)isobutyramido-2-phényl-2',3',5'-triméthyl-4'-hydroxyglycylanilide.

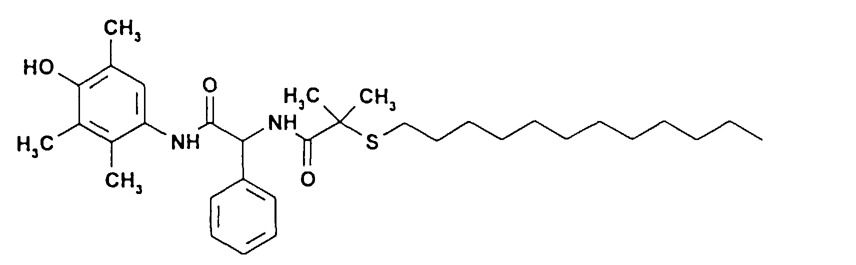

Ce composé a été préparé selon les procédés décrits dans les exemples 1a et 1b en utilisant le N-boc-phényl glycine puis selon le procédé de l'exemple 2.
F = 71°C
CCM = gel de silice 60F54Merck. Rf = 0,35 (CH₂Cl₂- AcOEt 90-10).

### EXEMPLE 11 :

### N-(α-n-dodécylthio)phénylacétamido-2',3',5'-triméthyl-4'-hydroxyglycylanilide.

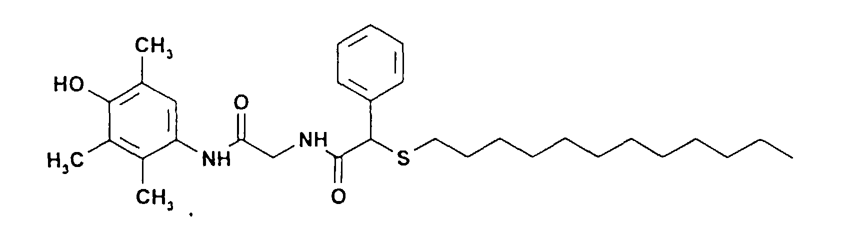

Ce composé a été obtenu selon le procédé décrit dans l'exemple 1 en utilisant le chlorure d'α-chloro-phenylacétyle.
F = 127°C
CCM : gel de silice 60F254 Merck. Rf = 0,58 (CH₂Cl₂- AcOEt 70-30).

### EXEMPLE 12 :

### N-(α-n-dodécylthio)isobutyramido-3',5'-bis-tertiobutyl-4'-hydroxyglycylanilide.

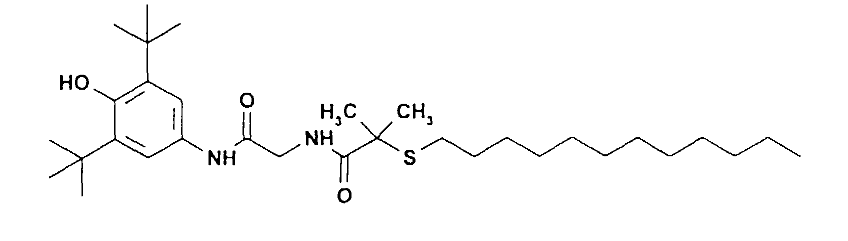

Ce composé a été obtenu selon le procédé décrit à l'exemple 2 en utilisant le 2,6-bisterbutyl-4-aminophénol.
F = 103°C
CCM : gel de silice 60F254 Merck. Rf = 0,63 (AcOEt - hexane 50-50).

### EXEMPLE 13 :

### N-(α-n-dodécylthio)propionamido-3',5'-bis-tertiobutyl-4'-hydroxyglycylanilide.

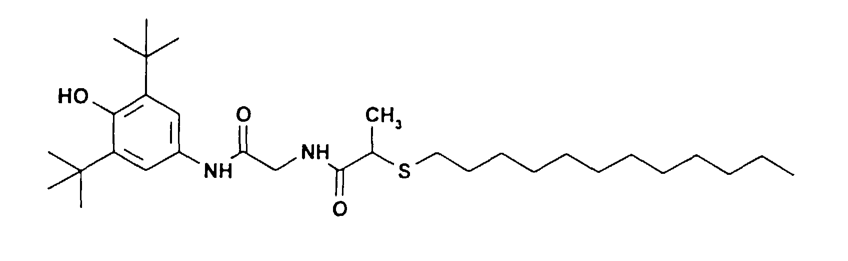

Ce composé a été obtenu selon l'exemple 12.
F = 93°C
CCM : gel de silice 60F254. Merck. Rf = 0,38 (AcOEt - hexane 50-50).

### EXEMPLE 14 :

### N-(α-n-dodécylthio)propionamido-3'-méthyl-4'-hydroxy-5'-tertiobutylglycylanilide.

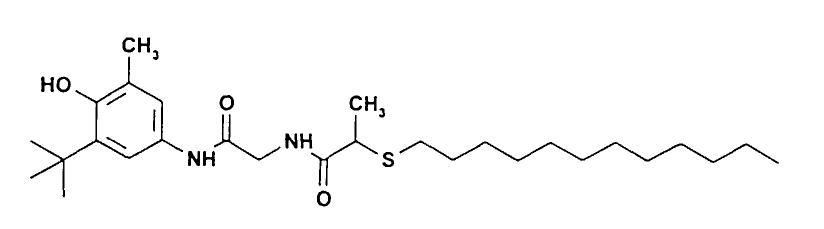

Ce composé a été obtenu selon le procédé décrit à l'exemple 1 en utilisant le 2-méthyl-4- amino-6-terbutyl-phénol.
F = 84°C
CCM : gel de silice 60F254 Merck. Rf = 0,42 (CH₂Cl₂- AcOEt 70-30).

### EXEMPLE 15 :

### N-méthyl-N-(α-n-dodecylthio)propionamido-3'-méthyl-4'-hydroxy-5'-tertiobutyl-glycylanilide.

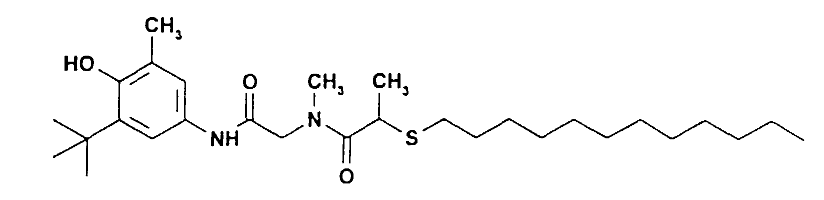

Ce composé a été obtenu selon l'exemple 14 en utilisant la N-boc-sarcosine.
F = 117°C
CCM : gel de silice 60F254 Merck. Rf = 0,28 (AcOEt - Hexane 50-50).

### EXEMPLE 16 :

### N-(α-n-dodécylthio)isobutyramido-3'-méthyl-4'-hydroxy-5'-tertiobutylglycylanilide.

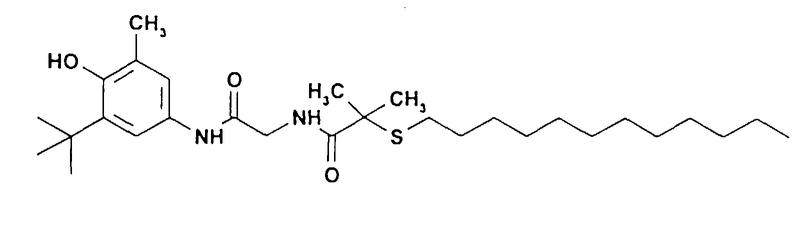

Ce composé a été préparé selon le procédé décrit à l'exemple 2.
F = 90°C
CCM : gel de silice 60F254 Merck. Rf = 0,38 (AcOEt - Hexane 50-50).

### EXEMPLE 17 :

### N-(α-n-dodécylthio)butyramido-3'-méthyl-4'-hydroxy-5'-tertiobutylglycylanilide.

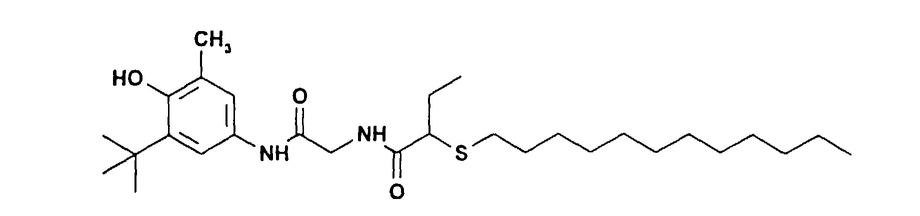

Ce composé a été obtenu selon le procédé décrit à l'exemple 1 en utilisant le bromure de 2-bromobutyryle.
F = 125°C
CCM : gel de silice 60F254 Merck. Rf = 0,42 (AcOEt - hexane 50-50).

Les composés de l'invention ont été soumis à des essais pharmacologiques qui ont montré leur intérêt potentiel dans le traitement de l'hypercholestérolémie et de la maladie athéromateuse.

Les composés ont été étudiés pour leur effet inhibiteur de l'ACAT et hypocholestérolémiant chez le rat d'une part et pour leur effet antioxydant d'autre part.

### 1) Inhibition de l'ACAT.

L'activité inhibitrice de l'ACAT (enzyme acyl CoA : cholestérol O acyltransférase) des composés a été évaluée in vitro en utilisant la technique de H. CHAUTAN et al. (Analytical Biochemistry 173, 436-439, 1988).

Les activités, exprimées en concentrations inhibitrices 50 % (CI₅₀), obtenues sur certains produits de l'invention sont reportées, à titre d'exemple, dans le tableau 3 suivant :

| Composé n° | CI₅₀ (µM) |
|---|---|
| 1 | 1,2 |
| 2 | 0,74 |
| 3 | 0,16 |
| 5 | 0,62 |
| 8 | 0,28 |
| CI 976 | 0,98 |
| DUP 128 | 0,09 |

### 2) Activité hypocholestérolémiante.

Des rats mâles (160-180 g) sont soumis pendant 4 jours à un régime hypercholestérolémique Altromin C 1061 et traités parallèlement par les composés en suspension dans la carboxy méthyl cellulose à 1 %, par voie orale.

Le 5ème jour, les animaux à jeun depuis 16 heures sont anesthésiés à l'éther éthylique, exsanguinés par prélèvement sur EDTA à l'aorte abdominale.

Le sang est immédiatement centrifugé et le plasma conservé à 4°C.

Le cholestérol plasmatique est alors dosé par la méthode CHOP-PAP (Boehringer - Mannheim Réf. 237574).

La dose efficace 50 (DE₅₀) correspond à la dose qui réduit de moitié la concentration en cholestérol plasmatique par rapport aux animaux témoins.

| Composé n° | DE₅₀ (mg/kg) |
|---|---|
| 1 | 1 |
| 2 | 3 |
| 3 | <2,5 |
| 5 | 2,5 |
| 8 4 | |
| CI 976 | 7,2 |
| DUP 128 | 1,4 |

### 3) Activité antioxydante.

### a) Péroxydation chimique.

En présence de Fe³⁺ et d'ADP, l'acide dihydroxyfumarique subit une autooxydation qui génère des radicaux libres oxygénés. Ces derniers entrainent la péroxydation des lipides microsomiaux hépatiques.

Cette péroxydation, effectuée sur des microsomes de foie de rat, est mesurée selon la technique à l'acide thiobarbiturique (formation de T.BARS) tel que décrite par S.Y.H. TSE et al. (Biochemical Pharmacology, Vol 42, n° 3, 459-464, 1991).

| Composé n° | CI₅₀ (µM) |
|---|---|
| 1 | 3 |
| 2 | 0,5 |
| 3 | 0,5 |
| 5 | 0,3 |
| 8 | 0,5 |
| CI 976 | > 10 |
| DUP 128 | > 10 |

### b) Oxydation des LDL.

Les LDL humaines (Sigma L 2139) sont oxydées par CuSO₄ 4,5 µM. Après une période d'incubation de 6 heures, la péroxydation est évaluée par mesure des T.BARS par spectrophotométrie à 532 nanomètres.

| Composé n° | CI₅₀ (µM) |
|---|---|
| 1 | 5 |
| 2 | 1 |
| 3 | 5 |
| 5 | 3 |
| 8 | 3 |
| CI 976 | 100 |
| DUP 128 | 30 |

Les composés de l'invention sont des hypocholestérolémiants inhibiteurs d'ACAT et antioxydants qui peuvent être utilisés pour le traitement des maladies telles que l'hypercholestérolémie et l'athérosclérose.

Les compositions pharmaceutiques peuvent être présentes sous la forme appropriée pour l'administration par voie orale, parentérale ou locale par exemple sous la forme de capsules, comprimés, granulés, gélules, solutés liquides, sirops ou suspensions buvables et contenir les excipients appropriés.

La posologie quotidienne peut aller de 10 à 2000 mg.

## Revendications

1. Nouveaux dérivés de glycylanilides caractérisés en ce qu'ils répondent à la formule générale I: 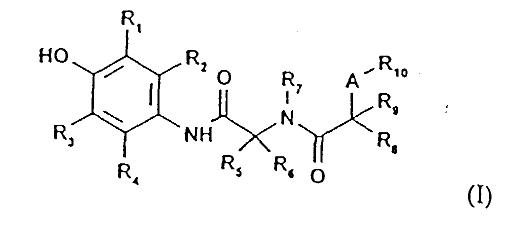 dans laquelle:
- R_{1,} R₂, R₃, R₄, R₇, identiques ou différents, représentent indépendamment les uns des autres l'hydrogène ou un radical alcoyle linéaire ou ramifié en C₁-C₄, à la condition toutefois qu'au moins trois des substituants R₁, R₂, R₃ et R₄ soient différents d'un atome d'hydrogène,
- R₅, R₆, R₈ et R₉, identiques ou différents, représentent indépendamment les uns des autres l'hydrogène, un radical alcoyle linéaire ou ramifié en C₁-C₄, ou un radical phényl éventuellement substitué,
- R₁₀ représente un radical alcoyle linéaire ou ramifié en C₃-C₁₆.,
- A représente un atome de soufre ou un groupement méthylène.

2. Composés répondant à la formule générale (I) selon la revendication 1, sélectionnés parmi le groupe suivant:
- N-(2-n-dodécylthio)propionamido-2',3',5'-triméthyl-4'-hydroxy-glycylanilide
- N-(α,α-diméthyl) tétradécanamido-2',3',5'- triméthyl-4'- hydroxy glycylanilide
- N-méthyl-N-(α-n-dodécylthio)isobutyramido-2',3',5'-triméthyl-4 '-hydroxy-glycylanilide
- N-(α-n-dodécylthio)-butyramido-2',3 ',5 '-triméthyl-4 '-hydroxy-glycylanilide
- N-(α-n-dodécylthio)isobutyramido-2',3 ',5 '-triméthyl-4 '-hydroxy-glycylanilide
- N-(α-n-dodécylthio)acétamido-2' ,3 ',5 '-triméthyl-4 '-hydroxy-glycylanilide
- N-(α-n-dodécylthio)butyramido-2-éthyl-2',3',5'-triméthyl-4 '-hydroxy-glycylanilide
- N-méthyl-N-(α-n-dodécylthio)propionamido-2',3',5'-triméthyl-4'-hydroxy-glycylanilide
- N-(α-n-dodécylthio)isobutyramido-2-n-butyl-2',3',5'-triméthyl-4'-hydroxy-glycylanilide
- N-(α-n-dodécylthio)isobutyramido-2-phényl-2',3',5'-triméthyl-4'-hydroxy-glycylanilide
- N-(α-n-dodécylthio)phénylacétamido-2',3 ,5 '-triméthyl-4 '-hydroxy-glycylanilide

3. Procédé de préparation des composés selon l'une des revendications 1 ou 2, caractérisé en ce que:
a) dans une première étape, on traite une aniline par un Boc-aminoacide en présence de chloroformate d'éthyle et de triéthylamine dans le diméthyl formamide puis par l'acide chlorhydrique 6 N pour fournir l'intermédiaire II 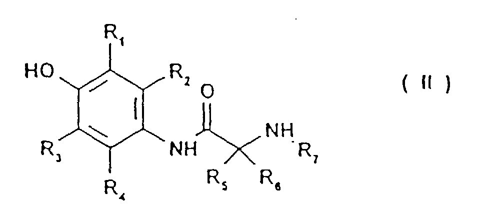 dans lequel R₁, R₂, R₃, R₄, R₅, R₆, R₇, sont tels que définis dans la revendication 1,
b) dans une deuxième étape, soit on traite l'intermédiaire II par un chlorure d'acide α halogéné III 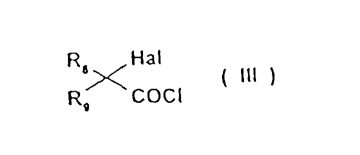 dans lequel R₈ et R₉ sont tels que définis dans la revendication 1 et Hal représente le chlore ou le brome,
puis par un thiol R₁₀SH, R₁₀ ayant la même signification que dans la revendication 1, en milieu sodium - éthanol ou tertiobutylate de potassium dans le tertiobutanol pour obtenir les composés I dans lesquels A = soufre, soit on traite l'intermédiaire II par un dérivé IV 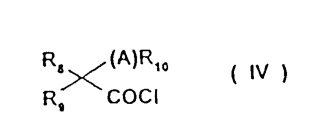 dans lequel R₈, R₉, R₁₀ et A ont la même signification que dans la revendication 1, dans l'acétate d'éthyle en présence de triéthylamine pour obtenir des composés dans lesquels A est un atome de soufre ou un groupement méthylène.

4. A titre de médicaments, les composés de formule générale I selon les revendications 1 ou 2, en particulier à titre de médicaments utilisés pour le traitement de maladies telles que l'hypercholestérolémie ou l'athérosclérose.

5. Compositions pharmaceutiques, caractérisées par le fait qu'elles contiennent, outre un support pharmaceutiquement acceptable, au moins un composé de formule générale I selon l'une quelconque des revendications 1 ou 2.

6. Utilisation de composés de formule générale I selon l'une quelconque des revendications 1 ou 2, pour la fabrication de médicaments destinés au traitement de maladies telles que l'hypercholestérolémie ou l'athérosclérose.

## Patentansprüche

1. Neue Derivate von Glycylaniliden der allgemeinen Formel I: 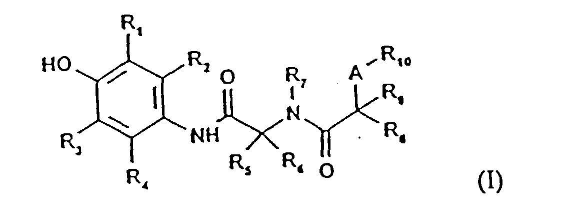
- in der R₁, R₂, R₃, R₄, R₇, unabhängig voneinander, für Wasserstoff oder eine lineare oder verzweigte Alkylgruppe mit 1 bis 4 Kohlstoffatomen stehen, unter der Bedingung, daß zumindest drei der Substituenten R₁, R₂, R₃ und/oder R₄ von Wasserstoff unterschiedlich sind,
- R₅, R₆, R₈ und R₉, unabhängig voneinander, Wasserstoff, eine geradkettige oder verzweigte Alkylgruppe mit 1 bis 4 Kohlstoffatomen oder eine gegebenenfalls substituierte Phenylgruppe sind,
- R₁₀ eine lineare oder verzweigte Alkylgruppe mit 3 bis 16 Kohlstoffatomen ist,
- A Schwefel oder eine Methylengruppe ist.

2. Verbindungen gemäß allgemeiner Formel (I), gemäß Anspruch 1 nämlich:
- N-(2-n-Dodecylthio)propionamido-2',3',5'-trimethyl-4'-hydroxy-glycylanilid;
- N-(α,α-Dimethyl)tetradecanamido-2',3',5'-trimethyl-4'-hydroxy glycylanilid;
- N-Methyl-N-(α-n-dodecylthio)isobutyramido-2','3',5'-trimethyl-4'-hydroxy-glycylanilid;
- N-(α-n-Dodecylthio)-butyramido-2',3',5'-trimethyl-4'-hydroxy-glycylanilid;
- N-(α-n-Dodecylthio)isobutyramido-2',3',5'-trimethyl-4'-hydroxy-glycylanilid;
- N-(α-n-Dodecylthio)acetamido-2',3',5'-trimethyl-4'-hydroxy-glycylanilid ;
- N-(α-n-Dodecylthio)butyramido-2ethyl-2',3',5'-trimethyl-4'-hydroxy-glycylanilid;
- N-Methyl-N-(α-n-dodecylthio)propionamido-2',3',5'-trimethyl-4'-hydroxy-glycylanilid;
- N-(α-n-Dodecylthio)isobutyramido-2-nbutyl-2',3',5'-trimetyl-4'-hydroxy-glycylanilid;
- N-(α-n-Dodecylthio)isobutyramido-2-phenyl-2',3',5'-trimethyl-4'-hydroxy-glycylanilid;
- N-(α-n-Dodecylthio)phenylacetamido-2',3',5'-trimethyl-4'-hydroxy-glycylanilid.

3. Verfahren zur Herstellung von Verbindungen gemäß einem der Ansprüche 1 oder 2, dadurch gekennzeichnet, daß man
a) in einer ersten Stufe ein Anilin mit einer Boc-Aminosäure in Gegenwart von Ethylchloroformat und Triethylamin in Dimethylformamid und dann mit Salzsäure 6N zur Zwischenverbindung II behandelt: 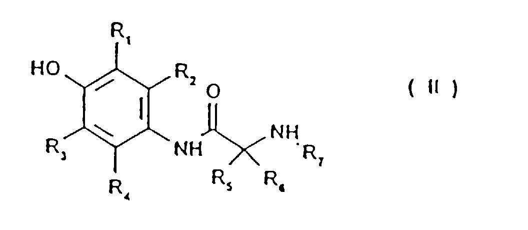 bei der R₁, R₂, R₃, R₄, R₅, R₆, R₇, die Bedeutungen gemäß Anspruch 1 besitzen,
b) in einer zweiten Stufe die Zwischenverbindung II mit einem α-halogenierten Säurechlorid III behandelt, 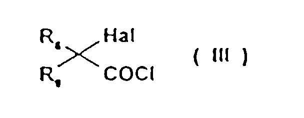 wobei R₈ und R₉ die Bedeutungen gemäß Anspruch 1 besitzen und Hal für Chlor oder Brom steht; dann mit einem Thiol R₁₀SH, wobei R₁₀ die Bedeutung gemäß Anspruch 1 besitzt, in einem Milieu Natrium-Ethanol oder Kalium-tert-butylat in tert-Butanol zum Erhalt der Verbindung I, in der A=Schwefel ist, behandelt oder die Zwischenverbindung II mit einem Derivat IV, 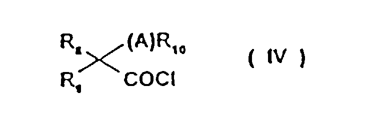 in dem R₈, R₉, R₁₀ und A die Bedeutungen gemäß Anspruch 1 besitzen, in Ethylacetat in Gegenwart von Triethylamin zum Erhalt der Verbindungen, in denen A Schwefel oder eine Methylengruppe ist, behandelt.

4. Verbindungen gemäß allgemeiner Form I gemäß Ansprüchen 1 oder 2 als Wirkstoff von Arzneimitteln, insbesondere zur Behandlung von Hypercholesterolemie oder Atherosclerose.

5. Pharmazeutische Zusammensetzungen, enthaltend mindestens eine Verbindung gemäß allgemeiner Formel I nach einem der Ansprüche 1 oder 2 zusammen mit einem pharmazeutisch verträglichen Träger.

6. Verwendung der Verbindungen der allgemeinen Formel I nach einem der Ansprüche 1 oder 2 zur Herstellung von Arzneimitteln zur Behandlung von Hypercholesterolemie oder Atherosclerose.

## Claims

1. New glycylanilide derivatives, characterized in that they correspond to the general formula I: 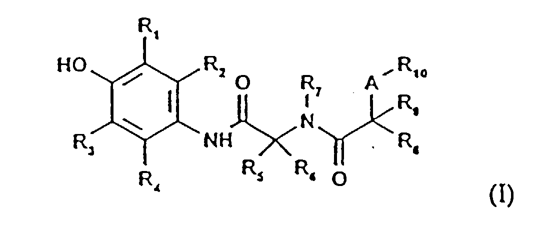 in which:
- R₁, R₂, R₃, R₄ and R₇, which may be identical or different, represent, independently of one another, hydrogen or a linear or branched C₁-C₄ alkyl radical, on condition, however, that at least three of the substituents R₁, R₂, R₃ and R₄ are other than a hydrogen atom,
- R₅, R₆, R₈ and R₉, which may be identical or different, represent, independently of one another, hydrogen, a linear or branched C₁-C₄ alkyl radical or an optionally substituted phenyl radical,
- R₁₀ represents a linear or branched C₃-C₁₆ alkyl radical,
- A represents a sulphur atom or a methylene group.

2. Compounds corresponding to the general formula (I) according to Claim 1, selected from the following group:
- N-(2-n-dodecylthio)propionamido-2',3',5'-trimethyl-4'-hydroxyglycylanilide
- N-(α,α-dimethyl)tetradecanamido-2',3',5'-trimethyl-4'-hydroxyglycylanilide
- N-methyl-N-(α-n-dodecylthio)isobutyramido-2',3',5'-trimethyl-4'-hydroxyglycylanilide
- N-(α-n-dodecylthio)butyramido-2',3',5'-trimethyl-4'-hydroxyglycylanilide
- N-(α-n-dodecylthio)isobutyramido-2',3',5'-trimethyl-4'-hydroxyglycylanilide
- N-(α-n-dodecylthio)acetamido-2',3',5'-trimethyl-4'-hydroxyglycylanilide
- N-(α-n-dodecylthio)butyramido-2-ethyl-2',3',5'-trimethyl-4'-hydroxyglycylanilide
- N-methyl-N-(α-n-dodecylthio)propionamido-2',3',5'-trimethyl-4'-hydroxyglycylanilide
- N-(α-n-dodecylthio)isobutyramido-2-n-butyl-2',3',5'-trimethyl-4'-hydroxyglycylanilide
- N-(α-n-dodecylthio)isobutyramido-2-phenyl-2',3',5'-trimethyl-4'-hydroxyglycylanilide
- N-(α-n-dodecylthio)phenylacetamido-2',3',5'-trimethyl-4'-hydroxyglycylanilide.

3. Process for preparing the compound according to either of Claims 1 and 2, characterized in that:
a) in a first step, an aniline is treated with a Boc-amino acid in the presence of ethyl chloroformate and triethylamine in dimethylformamide and then with 6N hydrochloric acid to yield the intermediate II. 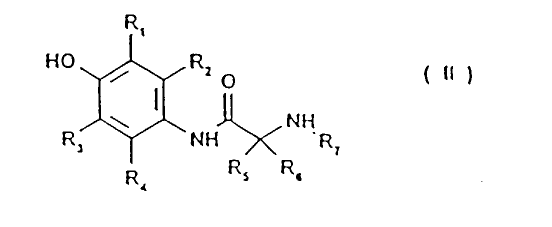 in which R₁, R₂, R₃, R₄, R₅, R₆ and R₇ are as defined in Claim 1.
b) in a second step, either the intermediate II is treated with an α-halogenated acid chloride III 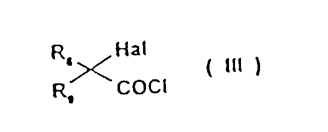 in which R₈ and R₉ are as defined in Claim 1 and Hal represents chlorine or bromine,
and then with a thiol R₁₀SH, R₁₀ having the same meaning as in Claim 1, in a sodium/ethanol medium or in a medium comprising potassium tert-butylate in tert-butanol, to obtain the compounds I in which A = sulphur, or the intermediate II is treated with a derivative IV 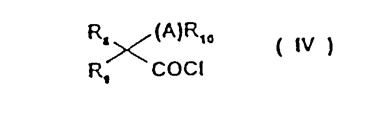 in which R₈, R₉, R₁₀ and A have the same meaning as in Claim 1, in ethyl acetate in the presence of triethylamine, to obtain compounds in which A is a sulphur atom or a methylene group.

4. As medicaments, the compounds of general formula I according to Claims 1 or 2, especially as medicaments used for the treatment of diseases such as hypercholesterolaemia or atherosclerosis.

5. Pharmaceutical compositions, characterized in that they contain, besides a pharmaceutically acceptable vehicle, at least one compound of general formula I according to either of Claims 1 and 2.

6. Use of compounds of general formula I according to either of Claims 1 and 2, for the manufacture of medicaments intended for the treatment of diseases such as hypercholesterolaemia or atherosclerosis.
